(19) Europäisches Patentamt
European Patent Office
Office européen des brevets

(11) **EP 2 056 124 B1**

(12) **EUROPEAN PATENT SPECIFICATION**

(45) Date of publication and mention
of the grant of the patent:
**11.05.2011 Bulletin 2011/19**

(51) Int Cl.:
*G01S 15/89* (2006.01)      *A61B 8/14* (2006.01)
*A61B 8/08* (2006.01)

(21) Application number: **07021153.7**

(22) Date of filing: **30.10.2007**

(54) **Aperture optimization for 3D ultrasound computer tomography**

Öffnungsoptimierung für 3D-Ultraschall-Computertomographie

Optimisation d'ouverture pour une tomographie informatique à ultrasons en 3D

(84) Designated Contracting States:
**AT BE BG CH CY CZ DE DK EE ES FI FR GB GR
HU IE IS IT LI LT LU LV MC MT NL PL PT RO SE
SI SK TR**

(43) Date of publication of application:
**06.05.2009 Bulletin 2009/19**

(73) Proprietor: **Karlsruher Institut für Technologie**
**76131 Karlsruhe (DE)**

(72) Inventors:
• **Gemmeke, Hartmut, Prof. Dr.**
**76297 Stutensee (DE)**
• **Schwarzenberg, Gregor**
**76133 Karlsruhe (DE)**
• **Ruiter, Nicole**
**76131 Karlsruhe (DE)**

(74) Representative: **Weddigen, Andreas**
**Karlsruher Institut für Technologie**
**Stabsabteilung Innovation**
**Postfach 36 40**
**76021 Karlsruhe (DE)**

(56) References cited:
**US-A1- 2006 106 307**

• **RUITER N V ET AL: "P3A-2 Resolution
Assessment of a 3D Ultrasound Computer
Tomograph Using Ellipsoidal Backprojection"
ULTRASONICS SYMPOSIUM, 2006. IEEE, IEEE,
PI, 1 October 2006 (2006-10-01), pages 1979-1982,
XP031076710 ISBN: 978-1-4244-0201-4**
• **SCHWARZENBERG G F ET AL: "Aperture
optimization for 3D ultrasound computer
tomography" 2007 IEEE ULTRASONICS
SYMPOSIUM IEEE PISCATAWAY, NJ, USA, 2007,
pages 1820-1823, XP002484732 ISBN:
978-1-4244-1383-6**

EP 2 056 124 B1

**Description**

At Forschungszentrum Karlsruhe a 3D ultrasound computer tomograph (USCT) for early breast cancer detection is currently under development [1]. The system setup consists of 384 emitting and 1536 receiving elements which are arranged in evenly spaced layers on a cylinder of 18 cm diameter and 15 cm height. For 3D image reconstruction synthetic aperture focusing technique (SAFT) is applied [2].

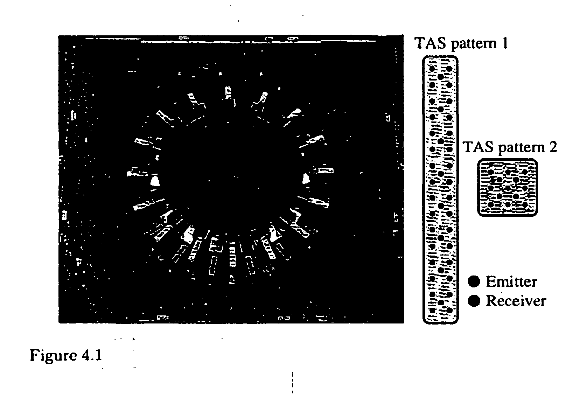

A previous study showed that the current cylindrical aperture has a major drawback for the vertical resolution [3], which makes it necessary to optimize the aperture. To derive an optimized aperture regarding the image quality of such a 3D imaging system several practical restrictions have to be considered. The number of transducers is limited due to available number of parallel channels of the data acquisition hardware. To be more cost effective the ultrasound transducers have to be grouped into so-called transducer array systems (TAS). The current TAS pattern (*TAS pattern 1*) is equipped with 8 emitters and 32 receivers (Fig. 4.1). As to the size of each transducer, there is a trade-off between the generated ultrasound energy and the opening angle. The current transducers have an opening angle of $\pm 15$ degree at -6 dB. The image quality in ultrasound imaging is also dependent on the imaged object. Geometric attenuation, breast attenuation and anisotropic scattering need to be considered. As a multi-static imaging system no analytical solution exists for deriving the theoretical point spread function ([4, 5]), why a numerical approach has to be developed.

The aim of this work is to derive an optimal aperture for USCT given an alternative TAS pattern (*TAS pattern 2*, Fig. 4.1) regarding image quality of this 3D imaging system and also taking as much physical reality into account as possible.

An optimal aperture for similar systems have been derived by [6, 7]. Both predicted a hemispherical aperture to be optimal in terms of resolution. Nevertheless ideal conditions were assumed in these studies such as point sources, no damping effects and ideal point scatterers.

For characterizing an imaging system the spatial point spread function (PSF) is typically analyzed, from which resolution and its spatial variance throughout the imaged region can be derived. Besides that the achievable contrast throughout the imaged region is of interest. Having finite sized transducer elements the emitted wave front of each transducer is not spherical but directed. As multi-static imaging is applied, not only the insonification fields of the emitters are of interest but also their intersection with the according sensing fields of the receivers, which results in a sensitivity field for each emitter-receiver-combination (ERC). The evaluation of this sensitivity is a third criteria as it delivers the basis of acquiring information from the imaged object. To evaluate these criteria, simulations of point scatterers in 3D are carried out for which realistic parameters are considered like geometric attenuation, sensor characteristic, breast attenuation and an angular defined scatterer profile.

Having 160 elements of *TAS pattern 2*, over 900 thousand A-scans can be recorded. The evaluation of the optimization criteria in 3D is computational complex.

To make this optimization problem solvable, the positioning of the TAS are restricted to a spheroidal base shape, so that only the according semi-axis are considered.

In the next section the methods are described for evaluating the imaging performance. Section 2.4 describes the constraints in more detail that were applied for making this optimization problem solvable followed by the optimization results.

**Chapter 2**

**Optimization criteria**

The aperture for the next generation of the USCT should be optimized with respect to isotropy and size of the 3D PSF, reduction of artifacts in the reconstructed images and overall sensitivity of all emitter-receiver pairs. To evaluate the resolution and reconstruction artifacts, i.e. the signal-to-artifact contrast, simulations of point scatterers are performed.

**2.1 Simulating measurements**

The analysis of the point spread function depends on the applied image reconstruction. With SAFT each recorded A-scan $A_{(i,j)}$ is backprojected to the image volume $I$ and summed up for each emitter $\bar{e}_i$ and receiver $\bar{r}_j$ from the set of all emitter-receiver-combinations $S_{ERC}$:

$$I(\vec{x}) = \sum_{(i,j) \in S_{ERC}} A_{(i,j)} \left( \frac{\|\bar{e}_i - \vec{x}\| + \|\bar{r}_j - \vec{x}\|}{v} \right), \qquad (2.1)$$

where $\bar{x}$ denotes the currently regarded voxel and $v$ the speed of sound.

Thus, the 3D image of a point scatterer is formed by a summation of spheroidal hulls of different widths and amplitudes.

The simulation of a point scatterer for one emitter-receiver-combination (ERC) begins with an initial pressure amplitude $p_0$ of the wavefront generated by the emitter. By traveling through space and interacting with the imaged object this amplitude is damped. The most important parameters are the beam profile of the assembled transducers, geometric attenuation, breast attenuation and reflection profile of the point scatterer.

For the geometric attenuation the solid angle $\Omega$ is calculated regarding the covered distance $z$ of the echo and the radius $r$, that denotes the scatterer or voxel radius, respectively:

$$\Omega = 2 \cdot \pi \cdot (1 - \cos\frac{\omega}{2}), \omega = 2 \cdot \arctan(\frac{r}{z}) \qquad (2.2)$$

The angle dependent beam profile $S(\alpha)$ is approximated by the Fraunhofer approximation using the Fourier transform of the square transducer aperture of size d and wavelength A of the emitted pulse [8]:

$$S(\alpha) = \frac{\sin(X)}{X}, X = \frac{\pi}{\lambda} \cdot d \cdot \sin(\alpha) \qquad (2.3)$$

The attenuation factor of the breast calculated by the distance $z'$ within the breast and a damping factor of 0.8 dB/cm/MHz (approx. 0.09 nepers) for the damping coefficient $\alpha(\omega)$:

$$p(z') = p_0 \cdot e^{-a(\omega) \cdot z'} \qquad (2.4)$$

The reflections from a point scatterer are material dependent, especially the size of the point scatterer compared to the wavelength determines the angular scatterer profile. These parameters are regarded by using an implementation of a model proposed by Faran [9].

For evaluating the resolution and contrast performance several positions throughout the breast are analyzed. To determine the sensitivity, the whole breast volume is analyzed at a coarse resolution with each voxel taken as point scatterer.

## 2.2 Resolution

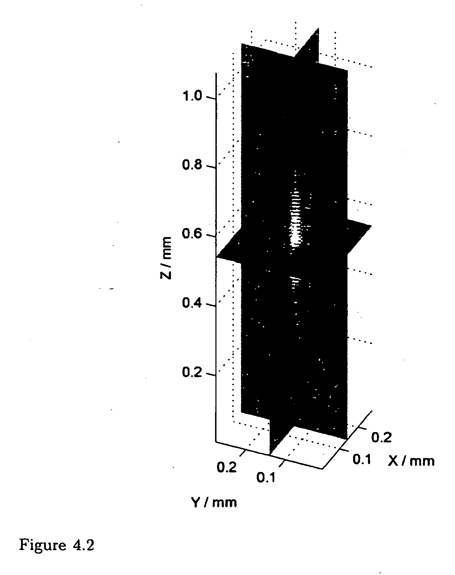

The resolution is evaluated by means of the 3D PSF, which is analyzed in the principal planes using the 2D full width at half maximum (FWHM) profile (Fig. 4.2). The PSF is determined geometrically as described in [3] for the 2D case and in [10] in 3D. A small and isotropic PSF is desirable. The measure to be minimized can be defined as the mean size $\bar{\rho}$ (small PSF) and the standard deviation $\sigma_\rho$ (isotropy) of the 2D FWHM profiles $\rho_{xy}$, $\rho_{xz}$ and $\rho_{yz}$ along the principal planes:

$$Res_{eval} = \bar{\rho} + \sigma_\rho, \rho = \{\rho_{xy}, \rho_{xz}, \rho_{yz}\} \qquad (2.5)$$

## 2.3 Contrast

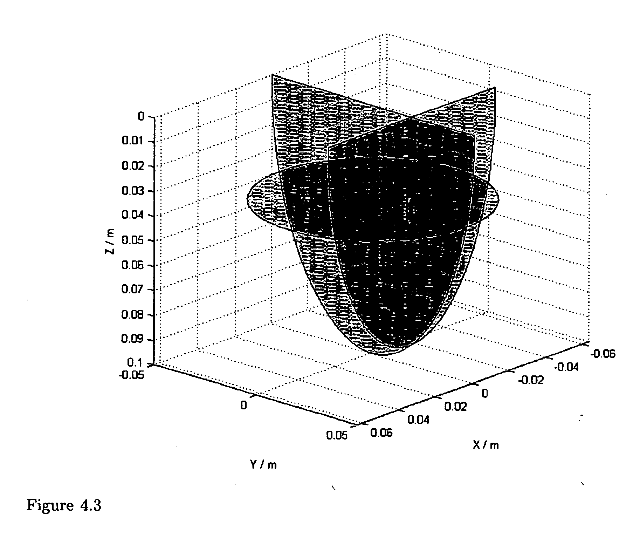

While the PSF analysis for the resolution evaluation is carried out only locally (local PSF), the contrast is assessed by reconstructing images of point scatterers for the whole breast (global PSF, Fig. 4.3). The spatially varying artifacts of a point reconstruction are analyzed in the rest of the image. The measure for the contrast is calculated by comparing the mean amplitude of the reconstructed point $\mu_{PSF}$ to the background artifacts evaluated as the mean $\mu_{BG}$ and standard deviation $\sigma_{BG}$ via the following contrast function, which is to be maximized:

$$SDNR = \frac{\mu_{PSF} - \mu_{BG}}{\sigma_{BG}} \qquad (2.6)$$

### 2.3.1 Sensitivity

The sensitivity assessment quantifies the acquired amount of information of a regarded volume. The ultrasound transducers are of finite size, so the insonification of one emitter is restricted by its opening angle in the direction of the emitter normal. For a receiver of finite size its beam profile describes a sensing field from where it receives echoes. To determine the overall sensitivity using all ERCs, insonification fields $E_i$ from the emitters and sensing fields from the receivers $R_j$ are computed in 3D regarding the implemented attenuation parameters. For each ERC these fields are multiplied resulting in a sensitivity field, which are summed up for each ERC:

$$sens = \sum_{(i,j) \in S_{ERC}} E_i \cdot R_j \qquad (2.7)$$

### 2.4 Constraints

For the further analysis in this work the angular reflection profile of the point scatterer is set to be uniform to reduce the parameter space. Phase aberrations caused by missing calibration or time-of-arrival detection are also neglected.

### 2.5 Semi-spheroidal region of interest

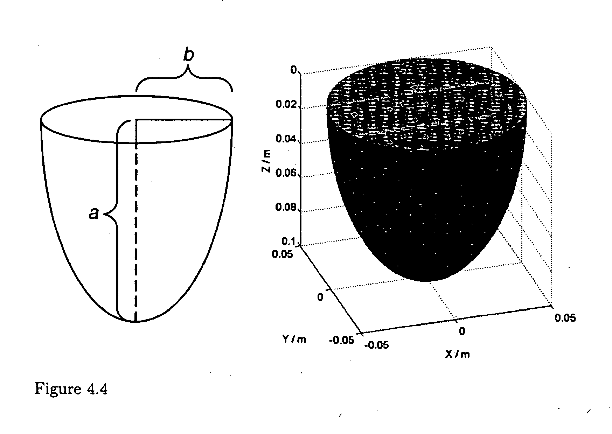

The evaluation of the optimization criteria is restricted to a semi-spheroidal region of interest (ROI) shaped like a breast (Fig. 4.4 left). This has the advantage that the traveled distance for each ERC and point scatterer can be calculated by line intersections with the spheroid. The major axis a of the ROI is set to 10 cm, the minor axis $b$ to 5 cm which corresponds to a breast volume of approx. 0.5 1 ('B'- to 'C'-cup).

For the contrast and resolution assessment 42 point scatterers are uniformly distributed throughout the breast volume (Fig. 4.4 right).

### 2.6 Data set reduction

The evaluations for the local and global PSF are very time consuming, as for every of the 42 positions the whole data set of approx. 900,000 ERCs has to be considered. As recommended in [6] not the full data set is processed with multi-static SAFT as this blurs the image. Because of this and also to speed up the calculations the data set for the local PSF analysis is reduced to one third, resulting in a computation time of approx. 90 s per point scatterer position (Pentium IV, 3.2 GHz). The image reconstructions for the evaluation of the global PSF are even more computational expensive as each A-scan is projected to every voxel. Having a voxel size of approx. 0.4 mm about 200,000 voxels have to be considered for all three imaging planes. So the data set for this evaluation is reduced to 30,000 A-scans. In order to use the most significant contributions those A-scans are chosen which have the highest amplitude.

### 2.7 TAS positioning

The calculation of the three measures in 3D is computational very expensive. These have to be carried out for each alteration of any TAS position or orientation. Thus the parameter space for possible TAS positionings is restricted to a spheroidal shell. The spheroidal shape as a generalization of the hemisphere gives much variety of possible apertures such as a bowl-shaped as well as cone-shaped one. This reduces the search space to two dimensions (two semi-axes). The TAS elements are equally distributed for each size of the spheroidal aperture.

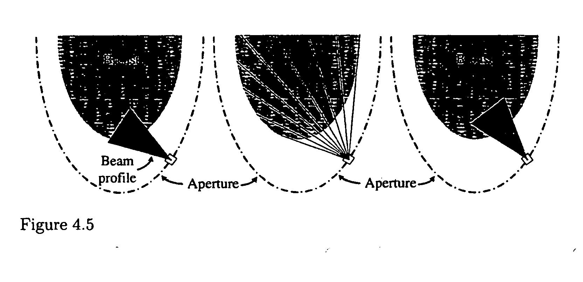

Due to the beam profile of the transducers the normal of the TAS has to be optimized. This is done automatically for each point in the parameter space (Fig. 4.5). Initially beginning from a TAS normal defined by the spheroid all possible normals which hit the breast are analyzed. Each normal is evaluated in terms of maximizing the insonification of the whole ROI. Crucial parameters which influence the amount of insonification within the breast are the beam profile and its orientation as well as the incident angles.

**Chapter 3**

**Results**

**3.1 Parameter space evaluation**

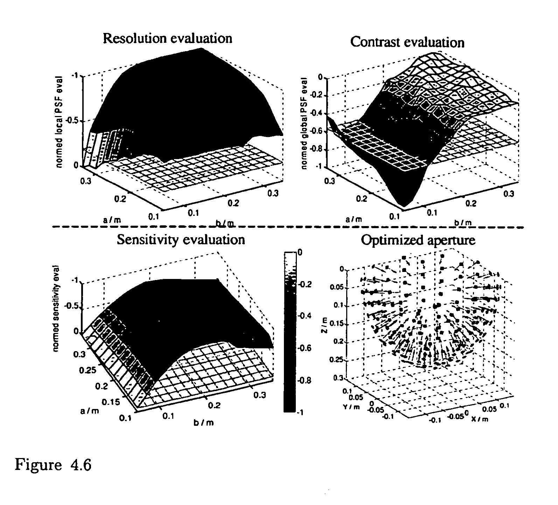

A first coarse evaluation of the parameter space has been carried out for each optimization criterion (Fig. 4.6). Beginning from the minimal size of a (10 cm) and *b* (5 cm) determined by the breast size, apertures for different semi-axes were evaluated at 2 cm steps up to 35 cm. This gives an overview over the trend for each of the optimization criteria. The extrema are used to normalize the results for the regarded parameter space. For comparison the plane mesh plot shows the evaluation of each criteria for the current USCT setup. High negative values are of interest as all optimization criteria are minimized.

For a high resolution the spheroidal aperture should be significantly larger than the imaged breast (> 20 cm for each semi-axis). Compared to the current cylindrical aperture the measure is up to 44% better showing the improved resolution.

The results for the contrast evaluation are ambivalent compared to the current cylindrical aperture. For minor axes below 10 cm the spheroidal aperture is higher rated than the cylindrical, but degrades fast for larger apertures.

According to the sensitivity the aperture should be significantly larger than the current cylinder and shaped like a bowl (a = 0.11 m, *b* = 0.20 m). This is due to the small opening angle of the equipped transducers. The greater the distance between breast and transducer, the better the insonification of the breast.

**3.2 Gradient-based optimization**

An optimization run using a gradient-based optimization scheme and equal weighting of the optimization criteria resulted in parameters a = 23.7 cm and *b* = 17.0 cm (Fig. 4.6 bottom right). Regarding the resolution, the size and deformations of the PSF throughout the ROI is reduced by 40% with this new spheroidal aperture. The sensitivity throughout the breast volume is almost 3 times higher than for the current cylindrical aperture. The contrast on the other hand is degraded resulting in an increase of the reconstruction artifacts by 13%.

Artifacts in the reconstructions are caused by the fact that both presented apertures are sparse. Backprojected echoes sum up at the locations of the point scatterers while causing artifacts in the rest of the image. These artifacts are reduced by destructive interference of backprojections arising from nearby transducers. *TAS pattern 1* is locally denser than *TAS pattern 2*, so more neighboring elements are available.

**Chapter 4**

**Discussion and future work**

The proposed restriction of the TAS positioning to a spheroidal shell makes this high-dimensional optimization problem solvable. An additional optimization in the vertical TAS orientation automatically ensures a good sensitivity. Compared to the current cylindrical aperture, the spheroidal aperture has major advantages regarding the resolution and insonification of the breast. By a non-equal weighting of the optimization criteria, an aperture can be derived which complies with the desired requirements, e.g. an aperture, that mainly decreases the reconstruction artifacts or mainly causes smaller and more isotropic PSFs.

So far no phase aberrations are considered that are caused by a missing calibration of the aperture, time of arrival detection, incorrect speed of sound within the breast and the water, etc. To improve the simulations further, parameters can be included in the optimization process such as transducer size, phase aberrations and non-equal TAS distribution. For the evaluation of the resolution and contrast a larger data set needs to be considered or the effects on each measure for reducing the data set remain to be studied.

**Description of the figures**

Figure 4.1: Experimental 3D USCT and different TAS patterns. The left image shows the current cylindrical USCT setup equipped with 48 TAS. A schematic of the current TAS pattern (*TAS pattern 1*) is shown in the middle with 8 emitters and 32 receivers. An alternative TAS pattern (*TAS pattern 2*) is shown on the right consisting of 4 emitters and 9 receivers.

Figure 4.2: Assessment of resolution and contrast in the principal planes: Local point spread function. The resolution is evaluated by means of the shape and size of the 2D FHWM profiles (black lines) of the 3D PSF.

Figure 4.3: Assessment of resolution and contrast in the principal planes: global point spread function. The contrast is evaluated by global reconstruction of the global PSF.

Figure 4.4: Left: Definition of a spheroid with major axis a and minor axis *b*. Right: Breast model with 42 positions of the point scatterers used for the analysis of resolution and contrast.

Figure 4.5: Automatic TAS normal alignment. Left: Initially the beam profile is aligned with the normal of the spheroid. Middle: Possible normals for the current TAS; each normal is evaluated in terms of maximizing the insonification with the given beam profile and incident angles. Right: Realignment of the TAS after optimization.

Figure 4.6: Parameter space evaluation and optimized aperture. Shown are the evaluations for each optimization criteria over the parameter space $a \in [10,35]$ cm and $b \in [5, 35]$ cm at steps of 2 cm. The resulting values are mapped to the domain [-1,0]. The plane mesh plot indicates the evaluation values for the current USCT setup in this domain. At the bottom right the optimized aperture for an equal weight of the criteria is shown ($a = 23.7$ cm, $b = 17.0$ cm).

## Bibliography

[1] H. Gemmeke and N.V. Ruiter, "3D Ultrasound Computer Tomography for Medical Imaging," Nucl. Instr. Meth. 2007, in press.

[2] R. N. Thomson, "Transverse and longitudinal resolution of the synthetic aperture focusing technique," in Ultrasonics, 1984, pp. 9-15.

[3] G. F. Schwarzenberg, R. Stotzka, M. Zapf, H. Gemmeke, and N. V. Ruiter, "Resolution Assessment of a 3D Ultrasound Computer Tomograph Using Ellipsoidal Backprojection," in Ultrasonics Symposium, 2006. IEEE, 2006, pp. 1979-1982.

[4] S. J. Norton and M. Linzer, "Ultrasonic reflectivity imaging in three dimensions: Exact Inverse Scattering Solutions for Plane, Cylindrical and Spherical Apertures," in IEEE Transactions on biomedical engineering, vol. 28, no. 2, 1981, pp. 202-220.

[5] M. Soumekh, Synthetic Aperture Radar Signal Processing: with MAT-LAB Algorithms. John Wiley & Sons, 1999.

[6] S. J. Norton and M. Linzer, "Ultrasonic reflectivity imaging in three dimensions: Reconstruction with spherical transducer arrays," in Ultrasonic Imaging, 1979.

[7] B. Yang, J. Scheuing, "Cramer-Rao bound and optimum sensor array for source localization from time differences of arrival," in Proc. IEEE ICASSP 2005, vol. 4, 2005, pp. 961-964.

[8] J. W. Goodman, Introduction to Fourier Optics. San Francisco: McGraw-Hill, 1968.

[9] J. J. Faran, "Sound Scattering by Solid Cylinders and Spheres," The Journal of the Acoustical Society of America, vol. 23, no. 4, pp. 405-418, 1951.

[10] G. F. Schwarzenberg and N. V. Ruiter, "3D PSF analysis for arbitrary transducer geometries and SAFT-based image reconstruction," in SPIE's Internl. Symposium Medical Imagine 2008, in press.

## Claims

1. Method of aperture optimization for 3D ultrasound computer tomography, USCT, for breast cancer detection consisting of the following steps:

A. Simulating measurement:

- with synthetic aperture focussing technique, SAFT, each recorded A-scan $A_{(i,j)}$ is backprojected to an image volume I and summed up for each emitter $\bar{e}_i$ and receiver $\bar{r}_j$ from the set of all emitter-receiver-combination $S_{ERC}$:

$$I(\vec{x}) = \sum_{(i,j) \in S_{ERC}} A_{(i,j)} \left( \frac{\|\vec{e}_i - \vec{x}\| + \|\vec{r}_j - \vec{x}\|}{v} \right),$$

where $\vec{x}$ denotes the currently considered voxel and V the speed of sound,
- the simulation of a point scatterer for one emitter-receiver combination, ERC, begins with an initial pressure amplitude $p_0$ of the waveform generated by the emitter,
- for the geometric attenuation a solid angle $\Omega$ is calculated regarding a covered distance $z$ of an echo and a radius $r$, that denotes the scatterer or voxel radius, respectively:

$$\Omega = 2 \cdot \pi \cdot (1 - \cos \frac{\omega}{2}), \quad \omega = 2 \cdot \arctan(r/z),$$

- an angle dependent beam profile $S(\alpha)$ is approximated by the Fraunhofer approximation using the Fourier transform of the square transducer aperture of size d and wavelength $\lambda$ of the emitted pulse:

$$S(\alpha) = \frac{\sin(X)}{X}, \quad X = \frac{\pi}{\lambda} \cdot d \cdot \sin(\alpha),$$

- an attenuation factor of the breast is calculated by the distance $z'$ within the breast and a given damping factor for the damping coefficient $a(\omega)$:

$$p(z') = p_0 \cdot e^{-a(\omega) \cdot z'}.$$

B. Resolution:

- a measure to be minimized is defined as a mean size $\bar{\rho}$ and the standard deviation $\sigma_\rho$ of the 2D full width at half maximum, FWHM, profiles $\rho_{xy}$, $\rho_{xz}$ and $\rho_{yz}$ along principal planes of the 3 D point spread function, PSF:

$$\text{Res}_{eval} = \bar{\rho} + \sigma_\rho, \quad \rho = \{\rho_{xy}, \rho_{xz}, \rho_{yz}\}.$$

C. Contrast:

- a measure for the contrast is calculated by comparing a mean amplitude of a reconstructed point $\mu_{PSF}$ to background artefacts evaluated as a mean $\mu_{BG}$ and standard deviation $\sigma_{BG}$ via the following contrast function, to be maximized:

$$SDNR = \frac{\mu_{PSF} - \mu_{BG}}{\sigma_{BG}},$$

D. Sensitivity:

- to determine an overall sensitivity using all emitter receiver combinations, ERC, insonification fields $E_i$ from the emitters and sensing fields from the receivers $R_j$ are computed in 3D regarding implemented attenuation parameters, and for each ERC these fields are multiplied resulting in a sensitivity field, which are summed up for each ERC:

$$sens = \sum_{(i,j) \in S_{ERC}} E_i \cdot R_j .$$

2. Method according to claim 1 consisting in that for further analysis the angular reflection profile of the point scatterer is set to be uniform.

3. Method according to claim 1 or 2 consisting in that an evaluation of the optimization criteria is restricted to a semi-spheroidal region of interest, ROI, shaped like a breast.

4. Method according to claim 3 consisting in that for contrast and resolution assessment point scatterers are uniformly distributed throughout the breast volume.

5. Method according to one of the claims 1 to 4 consisting in that for a data set reduction not the full set is processed with SAFT.

6. Method according to claim 5 consisting in that the data set is reduced to a given number of A-scans.

7. Method according to claim 6 consisting in that to use the most significant contributions A-scans which have the highest amplitude are chosen.

8. Method according to one of the claims 1 to 7 consisting in that for a transducer array positioning, TAS, the TAS-elements are equally distributed for each size of spheroidal aperture.

9. Method according to claim 8 consisting in that the normal of a TAS will be automatically done for each point in the parameter space, initially beginning from a TAS-normal defined by a spheroid all possible normals are analyzed, which hit the breast whereas each normal is evaluated in terms of maximizing the insonification of the ROI.

**Patentansprüche**

1. Verfahren zur Optimierung der Apertur für 3D-Ultraschall Computertomographie, USCT, zur Erfassung von Brustkrebs, umfasst die folgenden Schritte:

A. Simulation einer Messung:

- mit Synthetischer-Apertur-Fokus-Technik, SAFT, wird jeweils ein aufgezeichneter A-Scan $A_{(i,j)}$ zu einem Bildwert I rückprojeziert und für jeden Sender $\vec{e}i$ und Empfänger $\vec{r}i$ aus der Menge aller Sender-Empfänger-Kombinationen $S_{ERC}$ aufsummiert:

$$I(\vec{z}) = \sum_{(i,j) \in S_{ERC}} A_{(i,j)} \left( \frac{\|\vec{e}_i - \vec{z}\| + \|\vec{r}_j - \vec{z}\|}{v} \right),$$

wobei $\vec{x}$ den jeweiligen Voxel und v die Geschwindigkeit des Schalls beschreiben.

- Die Simulation der Streuung eines Punktes für eine Sender-Empfänger-Kombination, ERC, beginnt mit einer anfänglichen Druckamplitude $p_0$ der Wellenform, die vom Sender erzeugt wird,
- für die geometrische Dämpfung wird ein Raumwinkel $\Omega$ bezüglich des aktuellen Abstands z eines Echos und eines Radius r berechnet, der den Streuungs- bzw. Voxel-Radius bezeichnet:

$$\Omega = 2 \cdot \pi \cdot (1 - \cos\frac{\omega}{2}), \omega = 2 \cdot \arctan(\frac{r}{z})\ ,$$

- eine winkelabhängige Richtcharakteristik $S(\alpha)$ wird durch die Fraunhofer-Näherung mit Hilfe der Fourier-Transformation der quadratischen Sensorapertur mit der Größe d und Wellenlänge $\lambda$ des emittierten Pulses angenähert:

$$S(\alpha) = \frac{\sin(X)}{X}, X = \frac{\pi}{\lambda} \cdot d \cdot \sin(\alpha)\ ,$$

- ein Dämpfungsfaktor der Brust wird über den Abstand z' innerhalb der Brust und einem gegebenen Dämpfungsfaktor für den Dämpfungskoeffizienten $a(\omega)$ berechnet:

$$p(z') = p_0 \cdot e^{-a(\omega) \cdot z'}\ .$$

B. Auflösung:

- die zu minimierende Größe ist definiert als ein Mittelwert $\bar{\rho}$ und der Standardabweichung $\sigma_\rho$ der 2D-Gesamtbreite-bei-halbem-Maximum-, FWHM, Profile $\rho_{xy}$, $\rho_{xz}$ und $\rho_{yz}$ entlang der Hauptebenen der 3D Punktverteilungsfunktion PSF:

$$Res_{eval} = \bar{\rho} + \sigma_\rho, \rho = \{\rho_{xy}, \rho_{xz}, \rho_{yz}\}\ .$$

C. Kontrast:

- eine Kenngröße des Kontrasts wird über den Vergleich einer mittleren Amplitude eines rekonstruierten Punkt $\mu_{PSF}$ zu Hintergrundartefakte als Mittelwert $\mu_{GG}$ und Standardabweichung $\sigma_{GB}$ ermittelt und über folgende Kontrastfunktion maximiert:

$$SDNR = \frac{\mu_{PSF} - \mu_{BG}}{\sigma_{BG}}\ ,$$

D. Empfindlichkeit:

- um eine allgemeine Empfindlichkeit zu ermitteln werden mit allen Sender-Empfänger-Kombinationen, ERC, *die* Ausleuchtungsfelder $E_i$ der Sender und die Erfassungsfelder der Empfängern $R_j$ unter Bechtung der Dämpfungsparameterin 3D berechnet und diese Felder für jedes ERC zu einem Empfindlichkeitsfeld multipliziert, und für jedes ERC aufsummiert:

$$SDNR = \frac{\mu_{PSF} - \mu_{BC}}{\sigma_{BC}}$$

.

**2.** Verfahren nach Anspruch 1, **dadurch gekennzeichnet dass** für die weitere Analyse des Winkelreflexionsprofils der Streupunkt einheitlich gesetzt wird.

**3.** Verfahren nach Anspruch 1 oder 2, **dadurch gekennzeichnet dass** sich eine Bewertung der Optimierungskriterien auf einen interessierenden halbellipsoiden Bereich, ROI, der wie eine Brust geformt ist, beschränkt.

**4.** Verfahren nach Anspruch 1, **dadurch gekennzeichnet dass** für die Beurteilung von Kontrast und Auflösung die Streupunkte uniform über das Brustvolumen verteilt sind.

**5.** Verfahren nach einem der Ansprüche 1 bis 4, **dadurch gekennzeichnet, dass** für eine Datensatz-Reduktion nicht der gesamte Satz ist mit SAFT verarbeitet wird.

**6.** Verfahren nach Anspruch 5, **dadurch gekennzeichnet dass** der Datensatz zu einer vorgegebenen Anzahl von A-scans reduziert wird.

**7.** Verfahren nach Anspruch 5, **dadurch gekennzeichnet dass** die am meist beitragenden A-Scans, die die höchste Amplitude aufweisen, ausgewählt werden.

**8.** Verfahren nach einem der Ansprüche 1 bis 7, **dadurch gekennzeichnet, dass** für die Positionierung der Wandlerarrays, TAS, die TAS-Elemente gleichermäßig für jede Größe der rotationsellipsoidalen Apertur verteilt sind.

**9.** Verfahren nach Anspruch 8, **dadurch gekennzeichnet dass** die Normalen eines TAS automatisch für jeden Punkt im Parameterraum ermittelt werden, wobei zunächst ausgehend von einer TAS-Normalen, definiert durch ein Rotationsellipsoid, alle mögliche Normalen analysiert werden, die auf die Brust auftreffen, wobei jede Normale bezüglich der Maximierung der *Ausleuchtung* der ROI ausgewertet wird.

**Revendications**

**1.** Procédé d'optimisation de l'ouverture en tomographie par ordinateur à ultrasons en trois dimensions 3D, USCT, pour la détection du cancer du sein comprenant les étapes suivantes :

A. mesure de simulation :

- en technique de focalisation d'ouverture de synthèse SAFT, chaque balayage-A enregistré A$_{(i,j)}$ est rétroprojecté sur un volume d'image I et est sommé pour chaque émetteur $\bar{e}_i$, et chaque récepteur $\bar{r}_j$ de l'ensemble de toutes les combinaisons émetteur/récepteur $S_{ERC}$ :

$$I(\vec{x}) = \sum_{(i,j)\in S_{ERC}} A_{(i,j)}\left(\frac{\|\vec{e}_i - \vec{x}\| + \|\vec{r}_j - \vec{x}\|}{\upsilon}\right),$$

dans laquelle,
$\bar{x}$ concerne le volume élémentaire voxel concerné et $v$ la vitesse du son,
- la simulation d'un point dispersé d'une combinaison émetteur/récepteur ERC, commence par une amplitude de pression initiale po de l'onde générée par l'émetteur,
- pour l'atténuation géométrique, on calcule un angle solide $\Omega$ concernant la distance couverte $z$ d'un écho et avec un rayon $r$ qui correspond au rayon dispersé ou rayon voxel respectif :

$$\Omega = 2 \cdot \pi \cdot (1 - \cos \frac{\omega}{2}), \ \omega = 2 \cdot \arctan \ (r/z),$$

- un angle dépendant du profil du faisceau $S(\alpha)$ est approché par l'approximation de Fraunhofer en utilisant la transformée de Fourier de l'ouverture de transducteur au carré de la dimension d et de la longueur d'onde $\lambda$ de l'impulsion émise :

$$S \ (\alpha) = \frac{sin \ (X)}{X}, X = \frac{\pi}{\lambda} \cdot d \cdot sin \ (\alpha),$$

- un coefficient d'atténuation du sein, est calculé par la distance $z'$ dans le sein et d'un coefficient d'atténuation donné pour le coefficient d'atténuation $\alpha \ (\omega)$ :

$$p \ (z') = p_0 \cdot e^{-a(\omega) \ z'} \ .$$

B. Résolution :

- on défini une mesure à minimiser comme dimension moyenne $\bar{\rho}$ et l'écart standard $\sigma_{\rho}$ de la largeur complète en 2D pour le demi maximum, FWHM, les profils $\rho_{xy}$ $\rho_{xz}$ et $\rho_{yz}$ suivant les plans principaux de la fonction de dispersion (PSF) en 3D :

$$\mathrm{Res}_{eval} = \bar{\rho} + \sigma_{\rho}, \ \rho = \{\rho_{xy}, \ \rho_{xz}, \ \rho_{yz}\}$$

C. Contraste :

- on calcule une mesure du contraste en comparant une amplitude moyenne d'un point reconstruit $\mu PSF$ par rapport aux artifices d'arrière-plan évalués comme moyenne $\mu_{BG}$ et comme écart standard $\sigma_{BG}$ par la fonction de contraste suivante à rendre maximale :

$$SDNR = \frac{\mu_{PSF} - \mu_{BG}}{\sigma_{BG}}$$

D. Sensibilité :

- pour déterminer la sensibilité globale en utilisant toutes les combinaisons émetteur/récepteur ERC, les champs d'insonification $E_i$ des émetteurs et les champs de détection des récepteurs $R_j$, sont calculées en paramètres d'atténuation en 3D et pour chaque combinaison ERC, on multiplie les champs résultant dans un champ de sensibilité et on fait la somme pour chaque combinaison ERC :

$$sens = \sum_{(i,j) \in S_{ERC}} E_i . R_j .$$

2.  Procédé selon la revendication 1, selon lequel

pour la suite de l'analyse, on rend uniforme le profil de réflexion angulaire du point dispersé.

3. Procédé selon la revendication 1 ou 2,
   selon lequel
   on évalue les critères d'optimisation en se limitant à une région considérée, semi-sphérique ROI, en forme de sein.

4. Procédé selon la revendication 3,
   selon lequel
   pour les points de fixation de contraste et de résolution sont répartis uniformément dans le volume du sein.

5. Procédé selon l'une des revendications 1 à 4,
   selon lequel
   pour une réduction d'un jeu de données, l'ensemble du jeu n'est pas traité par SAFT.

6. Procédé selon la revendication 5,
   selon lequel
   le jeu de données est réduit à un nombre donné de balayages-A.

7. Procédé selon la revendication 6,
   selon lequel
   pour utiliser les contributions les plus significatives, on choisit les balayages A qui ont l'amplitude la plus élevée.

8. Procédé selon l'une des revendications 1 à 7,
   selon lequel
   pour un positionnement de réseau transducteur TAS, les éléments TAS sont répartis de manière égale pour chaque dimension d'ouverture sphéroïdale.

9. Procédé selon la revendication 8,
   selon lequel
   la normale au positionnement TAS sera faite automatiquement pour chaque point dans l'espace des paramètres en commençant à partir d'un TAS normal défini par un sphéroïde dont on analyse toutes les normales possibles et qui rencontre le sein alors que chaque normale est évaluée en terme de maximisation de l'insonification de ROI.

TAS pattern 1

TAS pattern 2

● Emitter
● Receiver

Figure 4.1

Figure 4.2

Figure 4.3

Figure 4.4

Figure 4.5

Figure 4.6

## REFERENCES CITED IN THE DESCRIPTION

**Non-patent literature cited in the description**

- **H. Gemmeke ; N.V. Ruiter.** 3D Ultrasound Computer Tomography for Medical Imaging. *Nucl. Instr. Meth.,* 2007
- **R. N. Thomson.** Transverse and longitudinal resolution of the synthetic aperture focusing technique. *Ultrasonics,* 1984, 9-15
- **G. F. Schwarzenberg ; R. Stotzka ; M. Zapf ; H. Gemmeke ; N. V. Ruiter.** Resolution Assessment of a 3D Ultrasound Computer Tomograph Using Ellipsoidal Backprojection. *Ultrasonics Symposium, 2006,* 2006, 1979-1982
- **S. J. Norton ; M. Linzer.** Ultrasonic reflectivity imaging in three dimensions: Exact Inverse Scattering Solutions for Plane, Cylindrical and Spherical Apertures. *IEEE Transactions on biomedical engineering,* 1981, vol. 28 (2), 202-220
- **M. Soumekh.** Synthetic Aperture Radar Signal Processing: with MAT-LAB Algorithms. John Wiley & Sons, 1999
- **S. J. Norton ; M. Linzer.** Ultrasonic reflectivity imaging in three dimensions: Reconstruction with spherical transducer arrays. *Ultrasonic Imaging,* 1979
- **B. Yang ; J. Scheuing.** Cramer-Rao bound and optimum sensor array for source localization from time differences of arrival. *Proc. IEEE ICASSP 2005,* 2005, vol. 4, 961-964
- **J. W. Goodman.** Introduction to Fourier Optics. McGraw-Hill, 1968
- **J. J. Faran.** ound Scattering by Solid Cylinders and Spheres. *Journal of the Acoustical Society of America,* 1951, vol. 23 (4), 405-418
- **G. F. Schwarzenberg ; N. V. Ruiter.** 3D PSF analysis for arbitrary transducer geometries and SAFT-based image reconstruction. *SPIE's Internl. Symposium Medical Imagine 2008,* 2008